# EUROPEAN PATENT SPECIFICATION

(11) **EP 0 966 482 B1**
(45) Date of publication and mention of the grant of the patent: **27.04.2005**
(21) Application number: 98901323.0
(22) Date of filing: 03.02.1998
(51) Int. Cl.: C07K 14/62, C07K 1/30

(54) **CRYSTALLISATION OF PROTEINS**
KRISTALLISATION VON PROTEINEN
CRISTALLISATION DE PROTEINES

(30) Priority: 07.02.1997 DK 14697
(43) Date of publication of application: 29.12.1999
(73) Proprietor: NOVO NORDISK A/S, 2880 Bagsvaerd (DK)
(72) Inventor: WHITTINGHAM, Jean, Lesley, York Y03 7NR (GB); BALSCHMIDT, Per, DK-3060 Espergaerde (DK)
(74) Representative: Jorgensen, Dan
(86) International application number: PCT/DK1998/000042
(87) International publication number: WO 1998/034953

(56) References cited:
- EP-A- 0 747 391
- GB-A- 2 290 294
- US-A- 3 856 771
- US-A- 4 959 351

## Description

### FIELD OF THE INVENTION

The present invention relates to a method of providing filterable crystals of zinc complexes of proinsulins, insulins and insulin analogues which have a lysine residue which carries a lipophilic substituent on the ε-amino group.

### BACKGROUND OF THE INVENTION

The isolation of pharmaceutical proteins in the crystalline state is important because crystals can be dried easily and subsequently stored at low temperature under conditions where the stability of the bulk protein is optimal. Insulins and insulin analogues in which the ε-amino group of a lysine residue contained therein has a lipophilic substituent *e.g.* in the form of an acyl group have a protracted profile of action and show promise for use in long-acting basal therapy in the treatment of IDDM (insulin-demanding diabetes mellitus) and NIDDM (non-insulin-demanding diabetes mellitus). The preparation of such acylated insulins and insulin analogues is described *i*.*a*. in Japanese patent application 1-254,699 (Kodama), in WO 95/07931 (Novo Nordisk), in EP 0 712 862 A2 (Eli Lilly) and in WO 96/29344 (Novo Nordisk). Unfortunately, such acylated insulins and insulin analogues have been found to be less prone to crystallize than the unmodified parent compounds.

Proinsulins, insulins and insulin analogues are rather labile proteins and their stability depends *i*.*a*. on the purity of the particular preparation. Optimal stability can be expected when a pure preparation is kept in solid form, in particular in the form of crystals. Unfortunately, precipitation of these compounds from solutions usually leads to amorphous precipitates which are difficult or impossible to isolate by filtration. In stead they can be isolated by centrifugation. However, when they are isolated by centrifugation it is difficult to free the particles efficiently from the mother liquor. Even when amorphous particles can be isolated by filtration, the amorphous material will usually be less pure than corresponding crystals because more impurities are embedded in amorphous material than in crystals.

The preparation of crystals of N^{εB29}-(myristoyl)des(B30) human insulin is described in European patent application No. 94926816.3 (EP 0 792290 B), technical information submitted for the file (Example 33). These zinc-free crystals were precipitated at pH 9 from 20% aqueous ethanol containing 0.625 M sodium chloride. A method for recovering acylated proteins, especially certain fatty acid-acylated insulins, by precipitation and filtration as a freely-flowing powder is described in EP 0 747 391 A2 (Eli Lilly). According to this method a filterable precipitate of a protein is obtained by adjusting the pH of an aqueous solution containing the protein and adding a suitable amount of alcohol. No formation of crystals is mentioned.

JL Wittigham et al. (*Biochemistry* **36** (1997) 2826 - 2831) describe crystallization of a zinc complex of Lys^{B29} tetradecanoyl des(B30) human insulin. It was found that the presence of phenol is essential for the crystallization.

For use in therapy, proteins must be prepared in highly purified form and the storage conditions must ensure that degradation during the storage is minimized. An acylated insulin or an acylated insulin analogue in highly purified form is administered in the form of a solution of a zinc complex thereof in a composition which further comprises a phenolic compound. Accordingly, it would be convenient in the production to store the bulk acylated insulin or acylated insulin analogue in the form of filterable crystals containing both insulin or insulin analogue, zinc and a phenolic compound.

It is thus an object of the present invention to provide a method by which zinc complexes of an acylated insulin or an acylated insulin analogue, also containing a phenolic compound, can be obtained in filterable, crystalline form.

According to the invention this object has been accomplished by precipitating the acylated insulin or acylated insulin analogue from an aqueous buffer.

### SUMMARY OF THE INVENTION

Thus, in its broadest aspect, the present invention relates to a method of providing zinc containing crystals of a protein derivative which is proinsulin, an insulin or an insulin analogue, each having a lysine residue which carries a lipophilic substituent on the ε-amino group, said method comprising
a) providing a solution of the derivative in an alkaline buffer which further contains a zinc salt and a phenol,
b) adjusting the pH value of the solution to a value between 7 and 10, and
c) isolating the crystals formed.

In a preferred embodiment, the protein derivative which has a lysine residue which carries a lipophilic substituent on the ε-amino group is a proinsulin, an insulin or an insulin analogue in which the ε-amino group of a lysine residue carries an acyl group.

In another preferred embodiment, the protein derivative is an insulin derivative selected from the group comprising N^{εB29}-(myristoyl)des(B30) human insulin, N^{εB29}-(myristoyl) human insulin, N^{εB29}-(palmitoyl) human insulin, N^{εB28}-(myristoyl)Lys^{B28}Pro^{B29} human insulin, N^{εB28}-(palmitoyl)Lys^{B28}Pro^{B29} human insulin, N^{εB30}-(myristoyl)Thr^{B29}Lys^{B30} human insulin, N^{εB30}-(palmitoyl)Thr^{B29}Lys^{B30} human insulin, N^{εB29}-(N-palmitoyl-γ-glutamyl)des(B30) human insulin, N^{εB29}-(N-lithocholyl-γ-glutamyl)des(B30) human insulin and N^{εB29}-(ω-carboxyheptadecanoyl)des(B30) human insulin.

In another preferred embodiment, an amount of phenol added which is from about 1.5% (w/w) to about 10% (w/w), preferably from about 1.5% (w/w) to about 3% (w/w) of the amount of protein present in the solution.

In another preferred embodiment, a phenol selected from the group comprising hydroxybenzene, *m*-cresol, methylparabene and ethylparabene is added.

In another preferred embodiment, the lipophilic group on the ε-amino group of the lysine residue is an acyl group having from 4 to 40, more preferred from 10 to 40 carbon atoms.

In another preferred embodiment, the lipophilic group on the ε-amino group of the lysine residue is a straight chain acyl group.

In another preferred embodiment, the buffer is composed of ammonia or an amine and an acid.

In another preferred embodiment, the buffer is composed of ammonia and phosphoric acid.

In another preferred embodiment, the buffer is composed of ammonia and a carboxylic acid.

In another preferred embodiment, the buffer is composed of an amine and phosphoric acid.

In another preferred embodiment, the buffer is composed of an amine and a carboxylic acid.

In another preferred embodiment, when the buffer is composed of an amine and an acid, the amine is selected from the group comprising tris(hydroxymethyl)aminomethane, 2-amino-2-methyl-1,3-propanediol, 2-hydroxyethylamine and tris(2-hydroxyethyl)amine.

In another preferred embodiment, the buffer is composed of an ampholytic compound, preferably aspartic acid or N-tris(hydroxymethyl)methylglycine, and optionally an acid.

In another preferred embodiment, when the buffer is composed of ammonia and a carboxylic acid or an amine and a carboxylic acid, the carboxylic acid is selected from the group comprising acetic acid, citric acid, lactic acid, malic acid, malonic acid, succinic acid, tartaric acid, tartronic acid and tricarballylic acid.

In another preferred embodiment, the concentration of the ammonia or amine in the buffer is between 0.1 M and 1 M, preferably between 0.2 M and 0.6 M.

In another preferred embodiment, the concentration of the phosphoric acid or the carboxylic acid in the buffer is between 0.05 M and 0.5 M, preferably between 0.05 M and 0.2 M.

In another preferred embodiment, the zinc salt added to the solution is zinc chloride or a zinc salt of a carboxylic acid, preferably a zinc salt of one the following acids: acetic acid, citric acid, lactic acid, malic acid, malonic acid, succinic acid, tartaric acid, tartronic acid and tricarballylic acid.

In another preferred embodiment, the zinc salt is added in a molar amount which is between 33% and 150% of the molar amount of the peptide monomer.

In another preferred embodiment, wherein the protein derivative which has a lysine residue which carries a lipophilic substituent on the ε-amino group is a proinsulin, an insulin or an insulin analogue in which the ε-amino group of a lysine residue carries an acyl group, the pH value of the solution in which the precipitation of crystals is performed is adjusted to a value in the range from about 8.0 to about 8.5.

### DETAILED DESCRIPTION OF THE INVENTION

### Definitions

As used in the present text the designation "insulin" is used to designate any naturally occurring insulin. The designation "insulin analogue" is used to designate a peptide with insulin activity, formally derived from a naturally occurring insulin by exchange of one or more amino acid residues and/or deletion of one or more amino acid residues and/or addition of one or more amino acid residues. An "acylated insulin" (or insulin analogue) is an insulin (or insulin analogue) which has an acyl group in the ε-amino group of a lysine residue contained in said insulin (or insulin analogue).

### Preferred embodiments

The precipitation of the crystals according to the present method is carried out in water which optionally contains a co-solvent. When a co-solvent is used, this is preferably a water-miscible solvent *e.g.* an alcohol.

The operations preceding the precipitation of the crystals are preferably carried out at a temperature around ambient. After standing from about 2 hours to about 40 hours at about ambient, the reaction mixture is cooled to a temperature near 0° C until no further precipitation of crystals occurs and the crystals are collected on a filter. If desired, the crystals can be washed with an ice-cold buffer solution corresponding to the solution from which the precipitation took place and with ice-cold ethanol.

The components of the buffer solution should preferably be less prone to form zinc complexes than insulin.

The present invention is further illustrated by the following examples which, however, are not to be construed as limiting the scope of protection as described in the appended claims.

### EXAMPLES

### Example 1

### Preparation of crystals of N^{εB29}-(myristoyl)des(B30) human insulin containing zinc and phenol.

Three stock solutions a), b) and c) were used during the preparation of crystals of N^{εB29}-(myristoyl)des(B30) human insulin. These solutions were prepared as follows:

### Stock solution a)

12.11 g of tris(hydroxymethyl)aminomethane was dissolved in 80 ml of water and the pH value of the solution was adjusted to 8.30 by means of approximately 7 ml of 5 N hydrochloric acid. Water was then added to a final volume of 100 ml.

### Stock solution b)

14.71 g of trisodium citrate dihydrate and 0.11 g of zinc acetate dihydrate were dissolved in water and 6.25 ml of a 3% (w/v) solution of phenol in water was added and the final volume of the solution adjusted to 100 ml.

### Stock solution c)

1.471 g of trisodium citrate dihydrate and 2.422 g of tris(hydroxymethyl)aminomethane were dissolved in water and the volume of the solution adjusted to 40 ml. The pH value was adjusted to 8.1 using 5 N hydrochloric acid and finally the volume was adjusted to 50 ml with water.

### Crystallization of N^{εB29}-(myristoyl)des(B30) human insulin

1.00 g of amorphous N^{εB29}-(myristoyl)des(B30) human insulin powder, obtained as described in WO 95/07931, was dispersed in a mixture of 38 ml of water and 2 ml of absolute ethanol and 40 ml of stock solution a) was added with stirring. When the insulin had dissolved 20 ml of stock solution b) was added and the pH value of the mixture was adjusted to be in the range 8.1-8.2 by addition of approximately 1 ml of 5 N hydrochloric acid. The mixture was left at ambient temperature with slow stirring overnight and then cooled to 4° C. The crystals formed were collected on a 50 mm filter and quickly washed with ice-cold stock solution c). After draining, the crystals were washed with 20 ml of ice-cold absolute ethanol and the drained crystals were dried *in vacuo.*

### Example 2

### Preparation of crystals of N^{εB29}-(myristoyl)des(B30) human insulin containing zinc and phenol.

The crystallization procedure according to Example 1 was repeated with use of 2-amino-2 methyl-1,3-propanediol in place of tris(hydroxymethyl)aminomethane.

### Example 3

### Preparation of crystals of N^{εB29}-(myristoyl)des(B30) human insulin containing zinc and phenol.

The crystallization procedure according to Example 1 was repeated with use of 2-hydroxyethylamine in place of tris(hydroxymethyl)aminomethane.

### Example 4

### Preparation of crystals of N^{εB29}-(myristoyl)des(B30) human insulin containing zinc and phenol.

The crystallization procedure according to Example 1 was repeated with use of ammonia in place of tris(hydroxymethyl)aminomethane.

### Example 5

### Preparation of crystals of N^{εB29}-(myristoyl)des(B30) human insulin containing zinc and phenol.

The crystallization procedure according to Example 1 was repeated with use of tris(2-hydroxyethyl)amine in place of tris(hydroxymethyl)aminomethane.

### Example 6

### Preparation of crystals of N^{εB29}-(myristoyl)des(B30) human insulin containing zinc and phenol.

The crystallization procedure according to Example 1 was repeated with use of N-tris(hydroxymethyl)methyl-glycine in place of tris(hydroxymethyl)aminomethane.

### Example 7

### Preparation of crystals of N^{εB29}-(myristoyl)des(B30) human insulin containing zinc and phenol.

The crystallization procedure according to Example 1 was repeated with use of L-aspartic acid in place of tris(hydroxymethyl)aminomethane.

### Example 8

### Preparation of crystals of N^{εB29}-(myristoyl)des(B30) human insulin containing zinc and phenol.

The crystallization procedure according to Example 1 was repeated with use of L-aspartic acid in place of both tris(hydroxymethyl)aminomethane and citric acid.

### Example 9

### Preparation of crystals of N^{εB29}-(myristoyl)des(B30) human insulin containing zinc and phenol.

The crystallization procedure according to Example 1 was repeated with use of sodium acetate in place of trisodium citrate.

### Example 10

### Preparation of crystals of N^{εB29}-(myristoyl)des(B30) human insulin containing zinc and phenol.

The crystallization procedure according to Example 1 was repeated with use of disodium tartrate in place of trisodium citrate.

### Example 11

### Preparation of crystals of N^{εB29}-(myristoyl)des(B30) human insulin containing zinc and phenol.

The crystallization procedure according to Example 1 was repeated with use of disodium succinate in place of trisodium citrate.

### Example 12

### Preparation of crystals of N^{εB29}-(myristoyl)des(B30) human insulin containing zinc and phenol.

The crystallization procedure according to Example 1 was repeated with use of disodium hydrogen phosphate in place of trisodium citrate.

### Example 13

### Preparation of crystals of N^{εB29}-(myristoyl)des(B30) human insulin containing zinc and phenol.

The crystallization procedure according to Example 1 was repeated with use of disodium malate in place of trisodium citrate.

### Example 14

### Preparation of crystals of N^{εB29}-(myristoyl)des(B30) human insulin containing zinc and phenol.

The crystallization procedure according to Example 1 was repeated with use of disodium malonate in place of trisodium citrate.

### Example 15

### Crystallization of N^{εB28}-(myristoyl)Lys^{B28}Pro^{B29} human insulin.

The crystallization procedure according to Example 1 was repeated with use of N^{εB28}-(myristoyl)Lys^{B28}Pro^{B29} human insulin in place of N^{εB29}-(myristoyl)des(B30) human insulin.

### Example 16

### Crystallization of N^{εB29}-(ω-carboxyheptadecanoyl)des(B30) human insulin.

The crystallization procedure according to Example 1 was repeated with use of N^{εB29}-(ω-carboxyheptadecanoyl)des(B30) human insulin in place of N^{εB29}-(myristoyl)des(B30) human insulin.

### Example 17

### Crystallization of N^{εB29}-(N-lithocholyl-γ-glutamyl)des(B30) human insulin.

The crystallization procedure according to Example 1 was repeated with use of N^{εB29}-(N-lithocholyl-γ-glutamyl)des(B30) human insulin in place of N^{εB29}-(myristoyl)des(B30) human insulin.

### Example 18

### Crystallization of N^{εB29}-(myristoyl)des(B30) human insulin

The crystallization method according to Example 1 was performed with use of sodium chloride in place of tris(hydroxymethyl)aminomethane following the procedure described below.

Two stock solutions d) and e) were used during the crystallization procedure. These solutions were prepared as follows:

### Stock solution d)

14.71 g of trisodium citrate dihydrate, 5.844 g of sodium chloride and 0.11 g of zinc acetate dihydrate were dissolved in about 75 ml of water and after addition of 6.25 ml of a 3% (w/v) solution of phenol in water the pH value was adjusted to 8.1 using 2 N sodium hydroxide and the final volume of the solution was adjusted to 100 ml.

### Stock solution e)

1.471 g of trisodium citrate dihydrate and 2.922 g of sodium chloride were dissolved in about 35 ml of water. The pH value was adjusted to 8.1 using 2 N sodium hydroxide and finally the volume was adjusted to 50 ml with water.

### Crystallization procedure:

1.00 g of amorphous N^{εB29}-(myristoyl)des(B30) human insulin powder was dispersed in a mixture of 78 ml of water and 2 ml of absolute ethanol and pH was adjusted to 8.3 with 0.1 M NaOH. When the insulin had dissolved 20 ml of a stock solution d) was added and the pH value of the mixture was adjusted to be in the range 8.1-8.2 by addition of 1 N hydrochloric acid. The mixture was slowly stirred overnight at ambient temperature and then cooled to 4° C. The crystals formed were collected on a 50 mm planar filter and quickly washed with ice-cold stock solution e). After draining, the crystals were washed with 20 ml of ice-cold absolute ethanol and the drained crystals were dried *in vacuo.*

## Claims

1. A method of providing zinc containing crystals of a protein which is a derivative of proinsulin, insulin or an insulin analogue which has a lysine residue which carries a lipophilic acyl group on the ε-amino group, said method comprising
a) providing a solution of the protein derivative in an alkaline buffer, which further contains a zinc salt and a phenol,
b) adjusting the pH value of the solution to a value between 7 and 10, and
c) isolating the crystals formed.

2. The method of claim 1 wherein the protein derivative is an insulin derivative selected from the group comprising N^{εB29}-(myristoyl)des(B30) human insulin, N^{εB29}-(myristoyl) human insulin, N^{εB29}-(palmitoyl) human insulin, N^{εB28}-(myristoyl)Lys^{B28}Pro^{B29} human insulin, N^{εB28}-(palmitoyl)Lys^{B28}Pro^{B29} human insulin, N^{εB30}-(myristoyl)Thr^{B29}Lys^{B30} human insulin, N^{εB30}-(palmitoyl)Thr^{B29}Lys^{B30} human insulin, N^{εB29}-(N-palmitoyl-γ-glutamyl)-des(B30) human insulin, N^{εB29}-(N-lithocholyl-γ-glutamyl)des(B30) human insulin and N^{εB29}-(ω-carboxyheptadecanoyl)des(B30) human insulin.

3. The method of claim 1 wherein the amount of phenol added is from about 1.5% (w/w) to about 10% (w/w), preferably from about 1.5% (w/w) to about 3% (w/w) of the amount of protein present in the solution.

4. The method of claim 1 wherein the phenol is selected from the group comprising hydroxybenzene, *m*-cresol, methylparabene and ethylparabene.

5. The method of claim 1 wherein the lipophilic group is an acyl group having from 4 to 40, more preferred from 10 to 40 carbon atoms.

6. The method of claim 1 wherein the lipophilic group is a straight chain acyl group.

7. The method of claim 1 wherein the alkaline buffer is a nitrogen-containing buffer.

8. The method of claim 7 wherein the buffer is composed of ammonia or an amine and an acid.

9. The method of claim 7 wherein the buffer is composed of ammonia and phosphoric acid.

10. The method of claim 7 wherein the buffer is composed of ammonia and a carboxylic acid.

11. The method of claim 7 wherein the buffer is composed of an amine and phosphoric acid.

12. The method of claim 7 wherein the buffer is composed of an amine and a carboxylic acid.

13. The method of claim 7 wherein the buffer is composed of an ampholytic compound, preferably aspartic acid or N-tris(hydroxymethyl)methylglycine, and optionally an acid.

14. The method of claim 11 or 12 wherein the amine is selected from the group comprising tris(hydroxymethyl)aminomethane, 2-amino-2-methyl-1,3-propanediol, 2-hydroxyethylamine and tris(2-hydroxyethyl)amine.

15. The method of claim 10 or 12 wherein the carboxylic acid is selected from the group comprising acetic acid, citric acid, lactic acid, malic acid, malonic acid, succinic acid, tartaric acid, tartronic acid and tricarballylic acid.

16. The method of claim 8 wherein the concentration of the ammonia or amine in the buffer is between 0.1 M and 1 M, preferably between 0.2 M and 0.6 M.

17. The method of claim 8 wherein the concentration of the phosphoric acid or the carboxylic acid in the buffer is between 0.05 M and 0.5 M, preferably between 0.05 M and 0.2 M.

18. The method of claim 1 wherein the zinc salt added to the solution is zinc chloride or a zinc salt of a carboxylic acid, preferably a zinc salt of one the acids mentioned in claim 15.

19. The method of claim 1 wherein the zinc salt is added in a molar amount which is between 33% and 150% of the molar amount of the peptide monomer.

20. The method of claim 1 wherein the pH value is adjusted to a value in the range from about 8.0 to about 8.5.

## Patentansprüche

1. Verfahren zum Bereitstellen zinkhaltiger Kristalle eines Proteins, das ein Derivat von Proinsulin, Insulin oder einem Insulinanalogon ist, welches einen Lysinrest aufweist, der einen lipophilen Acylrest an der ε-Aminogruppe trägt, wobei das Verfahren Folgendes umfasst
a) Bereitstellen einer Lösung des Proteinderivats in einem alkalischen Puffer, der ferner ein Zinksalz und ein Phenol enthält,
b) Einstellen des pH-Wertes der Lösung auf einen Wert zwischen 7 und 10 und
c) Isolieren der gebildeten Kristalle.

2. Verfahren nach Anspruch 1, wobei das Proteinderivat ein Insulinderivat ist, ausgewählt aus der Gruppe, umfassend N^{εB29}-(Myristoyl)des(B30)-Humaninsulin, N^{εB29}-(Myristoyl)-Humaninsulin, N^{εB29}-(Palmitoyl)-Humaninsulin, N^{εB28}-(Myristoyl)Lys^{B28}Pro^{B29}-Humaninsulin, N^{εB28}-(Palmitoyl)Lys^{B28}Pro^{B29}-Humaninsulin, N^{εB30}-(Myristoyl)Thr^{B29}Lys^{B30}-Humaninsulin, N^{εB30}-(Palmitoyl)Thr^{B29}Lys^{B30}-Humaninsulin, N^{εB29}-(N-Palmitoyl-γ-glutamyl)des(B30)-Humaninsulin, N^{εB29}-(N-Lithocholyl-γ-glutamyl)des(B30)-Humaninsulin und N^{εB29}-(ω-Carboxyheptadecanoyl)des(B30)-Humaninsulin.

3. Verfahren nach Anspruch 1, wobei die Menge des zugesetzten Phenols etwa 1,5 Gewichtsprozent bis etwa 10 Gewichtsprozent, vorzugsweise etwa 1,5 Gewichtsprozent bis etwa 3 Gewichtsprozent der Menge des in der Lösung vorliegenden Proteins beträgt.

4. Verfahren nach Anspruch 1, wobei das Phenol ausgewählt ist aus der Gruppe, umfassend Hydroxybenzol, *m*-Cresol, Methylparaben und Ethylparaben.

5. Verfahren nach Anspruch 1, wobei der lipophile Rest ein Acylrest ist, der 4 bis 40, stärker bevorzugt 10 bis 40 Kohlenstoffatome aufweist.

6. Verfahren nach Anspruch 1, wobei der lipophile Rest ein geradkettiger Acylrest ist.

7. Verfahren nach Anspruch 1, wobei der alkalische Puffer ein stickstoffhaltiger Puffer ist.

8. Verfahren nach Anspruch 7, wobei der Puffer aus Ammoniak oder einem Amin und einer Säure besteht.

9. Verfahren nach Anspruch 7, wobei der Puffer aus Ammoniak und Phosphorsäure besteht.

10. Verfahren nach Anspruch 7, wobei der Puffer aus Ammoniak und einer Carbonsäure besteht.

11. Verfahren nach Anspruch 7, wobei der Puffer aus einem Amin und Phosphorsäure besteht.

12. Verfahren nach Anspruch 7, wobei der Puffer aus einem Amin und einer Carbonsäure besteht.

13. Verfahren nach Anspruch 7, wobei der Puffer aus einer ampholytischen Verbindung, vorzugsweise Asparaginsäure oder N-Tris(hydroxymethyl)methylglycin, und wahlweise einer Säure besteht.

14. Verfahren nach Anspruch 11 oder 12, wobei das Amin ausgewählt ist aus der Gruppe, umfassend Tris(hydroxymethyl)aminomethan, 2-Amino-2-methyl-1,3-propandiol, 2-Hydroxyethylamin und Tris(2-hydroxyethyl)amin.

15. Verfahren nach Anspruch 10 oder 12, wobei die Carbonsäure ausgewählt ist aus der Gruppe, umfassend Essigsäure, Zitronensäure, Milchsäure, Apfelsäure, Malonsäure, Bernsteinsäure, Weinsäure, Tartronsäure und Tricarballylsäure.

16. Verfahren nach Anspruch 8, wobei die Konzentration des Ammoniaks oder Amins im Puffer zwischen 0,1 M und 1 M, vorzugsweise zwischen 0,2 M und 0,6 M liegt.

17. Verfahren nach Anspruch 8, wobei die Konzentration der Phosphorsäure oder der Carbonsäure im Puffer zwischen 0,05 M und 0,5 M, vorzugsweise zwischen 0,05 M und 0,2 M liegt.

18. Verfahren nach Anspruch 1, wobei das zur Lösung zugesetzte Zinksalz Zinkchlorid oder ein Zinksalz einer Carbonsäure, vorzugsweise ein Zinksalz einer der in Anspruch 15 genannten Säuren ist.

19. Verfahren nach Anspruch 1, wobei das Zinksalz in einer Molmenge zugesetzt wird, die zwischen 33% und 150% der Molmenge des Peptidmonomers beträgt.

20. Verfahren nach Anspruch 1, wobei der pH-Wert auf einen Wert im Bereich von etwa 8,0 bis etwa 8,5 eingestellt ist.

## Revendications

1. Un procédé d'obtention de cristaux renfermant du zinc d'une protéine qui est un dérivé de proinsuline, d'insuline ou d'un analogue d'insuline qui a un résidu lysine qui porte un groupe acyle lipophile sur le groupe ε-amino, ledit procédé comprenant :
a) la prévision d'une solution du dérivé de protéine dans un tampon alcalin, qui comporte, en outre, un sel de zinc et un phénol,
b) l'ajustement du pH de la solution à une valeur comprise entre 7 et 10 et
c) l'isolement des cristaux formés.

2. Le procédé selon la revendication 1, dans lequel le dérivé de protéine est un dérivé d'insuline choisi dans le groupe comprenant l'insuline humaine N^{εB29}-(myristoyl)des(B30), l'insuline humaine N^{εB29}-(myristoyle), l'insuline humaine N^{εB29}-(palmitoyle), l'insuline humaine N^{εB28}-(myristoyl)Lys^{B28}Pro^{B29}, l'insuline humaine N^{εB28}-(palmitoyl)Lys^{B28}Pro^{B29}, l'insuline humaine N^{εB30}-(myristoyl)Thr^{B29}Lys^{B30}, l'insuline humaine N^{εB30}-(palmitoyl)Thr^{B29}Lys^{B30}, l'insuline humaine N^{εB29}-(N-palmitoyl-γ-glutamyl)-des(B30), l'insuline humaine N^{εB29}-(N-lithocholyl-γ-glutamyl)des(B30) et l'insuline humaine N^{εB29}-(ω-carboxyheptadécanoyl)des(B30).

3. Le procédé selon la revendication 1, dans lequel la quantité de phénol ajouté se situe d'environ 1,5% (p/p) à environ 10% (p/p), de préférence d'environ 1,5% (p/p) à environ 3% (p/p) de la quantité de protéine présente dans la solution.

4. Le procédé selon la revendication 1, dans lequel le phénol est choisi dans le groupe comprenant l'hydroxybenzène, le m-crésol, le méthylparaben et l'éthylparaben.

5. Le procédé selon la revendication 1, dans lequel le groupe lipophile est un groupe acyle ayant de 4 à 40, mieux encore de 10 à 40 atomes de carbone.

6. Le procédé selon la revendication 1, dans lequel le groupe lipophile est un groupe acyle à chaîne droite.

7. Le procédé selon la revendication 1, dans lequel le tampon alcalin est un tampon contenant de l'azote.

8. Le procédé selon la revendication 7, dans lequel le tampon est constitué d'ammoniaque ou d'une amine et d'un acide.

9. Le procédé selon la revendication 7, dans lequel le tampon est constitué d'ammoniaque et d'acide phosphorique.

10. Le procédé selon la revendication 7, dans lequel le tampon est constitué d'ammoniaque et d'acide carboxylique.

11. Le procédé selon la revendication 7, dans lequel le tampon est constitué d'une amine et d'acide phosphorique.

12. Le procédé selon la revendication 7, dans lequel le tampon est constitué d'une amine et d'un acide carboxylique.

13. Le procédé selon la revendication 7, dans lequel le tampon est constitué d'un composé ampholytique, de préférence l'acide aspartique ou la N-tris(hydroxyméthyl)méthylglycine et éventuellement d'un acide.

14. Le procédé selon la revendication 11 ou 12, dans lequel l'amine est choisie dans le groupe comprenant le tris(hydroxyméthyl)aminométhane, le 2-amino-2-méthyl-1,3-propanediol, la 2-hydroxyéthylamine et la tris(-2-hydroxyéthyl)amine.

15. Le procédé selon la revendication 10 ou 12, dans lequel l'acide carboxylique est choisi dans le groupe comprenant l'acide acétique, l'acide citrique, l'acide lactique, l'acide malique, l'acide malonique, l'acide succinique, l'acide tartrique, l'acide tartronique et l'acide tricarballylique.

16. Le procédé selon la revendication 8, dans lequel la concentration de l'ammoniaque ou de l'amine dans le tampon se situe entre 0,1 M et 1 M, de préférence entre 0,2 M et 0,6 M.

17. Le procédé selon la revendication 8, dans lequel la concentration de l'acide phosphorique ou de l'acide carboxylique dans le tampon se situe entre 0,05 M et 0,5 M, de préférence entre 0,05 M et 0,2 M.

18. Le procédé selon la revendication 1, dans lequel le sel de zinc ajouté à la solution est du chlorure de zinc ou un sel de zinc d'un acide carboxylique, de préférence un sel de zinc d'un des acides mentionnés dans la revendication 15.

19. Le procédé selon la revendication 1, dans lequel le sel de zinc est ajouté selon une concentration molaire qui se situe entre 33 % et 150 % de la quantité molaire du monomère de peptide.

20. Le procédé selon la revendication 1, dans lequel le pH est ajusté à une valeur dans la gamme d'environ 8,0 à environ 8,5.
